# FASCICULE DE BREVET EUROPEEN

(11) **EP 1 494 880 B1**
(45) Date de publication et mention de la délivrance du brevet: **24.05.2006**
(21) Numéro de dépôt: 03746328.8
(22) Date de dépôt: 09.04.2003
(51) Int. Cl.: B60H 3/06

(54) **PURIFICATION DE L'AIR DE L'HABITACLE D'UN VEHICULE PAR ADSORPTION ET PHOTOCATALYSE**
REINIGUNG DER LUFT IN EINER FAHRGASTZELLE EINES FAHRZEUGS DURCH ADSORPTION UND PHOTOKATALYSE
PURIFICATION OF THE AIR IN A VEHICLE CABIN BY ADSORPTION AND PHOTOCATALYSIS

(30) Priorité: 12.04.2002 FR 0204647
(43) Date de publication de la demande: 12.01.2005
(73) Titulaire: VALEO SYSTEMES THERMIQUES, 78320 Le Mesnil Saint-Denis (FR)
(72) Inventeur: PAUMIER, Carine, F-78000 Versailles (FR); FEUILLARD, Vincent, F-78320 Le Mesnil Saint Denis (FR)
(86) Numéro de dépôt international: PCT/FR2003/001126
(87) Numéro de publication internationale: WO 2003/086792

(56) Documents cités:
- EP-A- 0 798 143
- EP-A- 0 960 644
- WO-A-01/62307
- WO-A-96/37281

## Description

### Arrière-plan de l'invention

La présente invention concerne la purification de l'air de l'habitacle d'un véhicule en traitant les gaz odorants ou nocifs par adsorption et photocatalyse.

De façon traditionnelle, dans un véhicule automobile, la purification de l'air de l'habitacle est assurée par des filtres à charbon actif ou plus généralement à adsorbant quelconque.

Les molécules des gaz polluants sont retenues par un phénomène d'adsorption sur la surface poreuse du charbon actif. Par la suite, une désorption ou relargage des polluants peut être effectuée sous certaines conditions de température.

La structure même de ces filtres impose une forte perte de charge initiale et un colmatage rapide qui se traduisent par une faible durée de vie, estimée à environ 20000 km.

Pour remédier à cet inconvénient, il est connu d'utiliser la photocatalyse qui consiste à épurer un fluide par l'action simultanée d'une ou plusieurs sources UV et d'un agent catalyseur tel que le dioxyde de titanium « TiO2» déposé sur un support.

Un agent photocatalyseur est un semi-conducteur et a donc comme tout matériau de ce type une bande de valence, une bande interdite et une bande de conduction.

Lorsqu'un photon, ayant une énergie suffisante par rapport à la largeur de la bande interdite, est absorbé par une molécule d'un agent photocatalyseur, une paire électron-trou est créée, plaçant la molécule dans un état excité. Cet état fait jouer à la molécule le rôle d'un catalyseur dans des réactions chimiques d'oxydation qui décomposent les polluants en vapeur d'eau « H2O » et en dioxyde de carbone « CO2 ».

En revanche, ces réactions chimiques sont lentes et, dans le cas où elles ne seraient pas complètes, produisent des sous-produits qui peuvent être aussi nocifs que les polluants de départ.

Un dispositif de purification de l'air conforme au préambule de la revendication 1 est divulgué dans le document WO 96/37281

### Objet et résumé de l'invention

L'invention a pour objet un dispositif de purification de l'air dont l'efficacité et la durée de vie sont augmentées et les rejets absents de toute nocivité. Un but de l'invention est aussi de réaliser un dispositif de purification écologique c'est-à-dire, dont les constituants sont formés de matériaux non toxiques et dont le nombre de pièces à remplacer durant la durée de vie d'un véhicule est très réduit.

Un autre but de l'invention est de réaliser un dispositif de purification de l'air réduit en volume tout en ayant une efficacité optimale en un seul passage de l'air à travers ce dispositif.

Ce but est atteint grâce à un dispositif de purification de l'air de l'habitacle d'un véhicule comprenant un filtre maintenu dans un cadre définissant une surface du filtre, disposé de manière à être traversé par un flux d'air et une source lumineuse irradiant la surface du filtre, caractérisé en ce que le filtre comprend une première couche de revêtement en fibres non tissées, une seconde couche de purification de l'air recouvrant la première couche et constituée d'un agent photocatalyseur intimement associé à des grains d'un adsorbant, et une troisième couche de revêtement en fibres non tissées recouvrant la seconde couche, l'ensemble de ces trois couches étant plissé en accordéon pour faire apparaître des ondulations ou plis qui augmentent la surface utile du filtre, et dont les génératrices des crêtes forment deux plans sensiblement parallèles et perpendiculaires au flux d'air.

L'invention est remarquable en ce que les polluants sont instantanément piégés par un adsorbant qui est constamment régénéré, augmentant ainsi sa durée de vie par 4 à 5 fois celle d'un filtre à charbon actif classique.

L'agent photocatalyseur peut être de l'oxyde de titanium tandis que l'adsorbant peut être du charbon actif, de la zéolite ou un mélange des deux.

De préférence, le charbon actif a une masse surfacique comprise entre 150 g/m2 et 450 g/m2 et le rapport en masse de l'oxyde de titanium par rapport au charbon actif est compris entre 1% et 20%.

Avantageusement, la lumière émise par la source lumineuse comporte une longueur d'onde comprise entre 200 nm et 400 nm.

De préférence, la source lumineuse et le filtre sont séparés par une distance comprise entre 2 mm et 30 mm. Lorsque cette source est montée à une distance d'environ 10 mm du filtre, elle assure une intensité lumineuse par unité de surface comprise entre 0,5 mW/cm2 et 10 mW/cm2.

La source lumineuse, irradiant le filtre, est constituée d'au moins un moyen d'émission lumineuse sans mercure ni plomb, qui est disposé en aval ou en amont du filtre par rapport au flux d'air et est orienté de sorte à irradier la majorité de la surface utile du filtre. Avantageusement, lorsque la source lumineuse est en amont du filtre, un filtre à particules comportant un agent photocatalyseur est monté en amont de la source lumineuse.

La source lumineuse peut comporter deux moyens d'émissions lumineuses.

Selon un mode de réalisation, le filtre comprend deux parties, séparées selon une section sensiblement perpendiculaire à la direction des génératrices et le moyen d'émission lumineuse est disposé entre ces deux parties, parallèlement à ladite section, et irradiant le filtre dans les deux sens de la direction des génératrices.

Selon un autre mode de réalisation, deux moyens d'émissions lumineuses sont disposés chacun de part et d'autre du côté du filtre perpendiculairement à la direction des génératrices, et irradiant le filtre dans la direction desdites génératrices.

Selon encore, un autre mode de réalisation, un moyen d'émission lumineuse est disposé sur un des côtés.

Selon un exemple de l'invention, le moyen d'émission lumineuse est une lampe UV tubulaire qui présente un lobe d'émission primaire orienté de sorte à irradier la majorité de la surface utile du filtre, et un lobe d'émission secondaire réfléchi vers la surface utile du filtre par un réflecteur.

Chaque lampe UV peut être alimentée par un transformateur connecté directement à la lampe sans l'intermédiaire de fils électriques.

Avantageusement, le moyen d'émission lumineuse comporte deux lampes UV alimentées par un transformateur unique.

Selon un aspect de l'invention, la ou les lampes, le ou les réflecteurs, et le ou les transformateurs sont montés dans un cadre externe destiné à recevoir le cadre support du filtre. Ce cadre externe comporte une longueur comprise entre 200 mm et 500 mm, une largeur comprise entre 100 mm et 300 mm et une épaisseur comprise entre 20 mm et 60 mm.

Selon d'autres exemples de invention, le moyen d'émission lumineuse peut être une lampe plate ou une plaque comportant une pluralité de diodes électroluminescentes.

Selon encore un autre exemple, le moyen d'émission lumineuse est une grille comportant une pluralité de diodes électroluminescentes sur ses noeuds, de façon à permettre le passage du flux d'air.

Selon un mode de réalisation, un filtre à particules est monté en amont du dispositif.

Selon un autre mode de réalisation, la première couche de revêtement est un filtre à particules, et le filtre combiné est disposé de manière à ce que cette première couche soit en amont de la troisième couche par rapport au flux d'air.

Avantageusement, un ioniseur est monté en amont du filtre à particules ou du filtre combiné.

Le dispositif de purification de l'air peut être implanté dans une installation de climatisation comportant une entrée d'air, un conduit, un groupe moto-ventilateur et un évaporateur.

Selon un mode de réalisation, le dispositif de purification de l'air est monté dans le conduit de l'installation de climatisation, entre le groupe moto-ventilateur et l'évaporateur.

Avantageusement, l'évaporateur sert de support pour un agent photocatalyseur, ce support étant irradié à partir des sources lumineuses montées en aval du dispositif de purification d'air.

Selon un autre mode de réalisation, le dispositif de purification de l'air est monté dans l'entrée d'air de l'installation de climatisation.

Selon encore un autre mode de réalisation, le dispositif de purification de l'air est monté dans un boîtier comportant un pulseur installé dans l'habitacle du véhicule.

L'invention a aussi pour but de fournir un procédé de réalisation d'un filtre, défini ci-avant, pour purifier l'air de l'habitacle d'un véhicule.

Ce but est atteint lorsqu'on associe intimement un agent photocatalyseur à des grains d'un adsorbant et on dépose le mélange ainsi formé entre deux couches de revêtement en fibres non tissées.

L'agent photocatalyseur peut être de l'oxyde de titanium, l'adsorbant peut être du charbon actif et de préférence on élabore un mélange selon une proportion en masse de l'oxyde de titanium par rapport au charbon actif comprise entre 1% et 20%.

Selon un procédé de réalisation, on applique un adhésif sur les faces internes des deux couches de revêtement avant de déposer le mélange entre elles et on applique une pression à froid sur l'ensemble ainsi formé.

Selon un autre procédé de réalisation, on compose ledit mélange on ajoutant un liant et on applique une pression à chaud sur l'ensemble ainsi formé.

Selon encore un autre procédé de réalisation, on applique un adhésif sur les faces internes des deux couches de revêtement avant de déposer séparément le titanium et le charbon actif et on applique une pression à froid ou à chaud sur l'ensemble ainsi formé.

### Brève description des dessins

D'autres particularités et avantages du dispositif et du procédé selon l'invention ressortiront à la lecture de la description faite ci-après, à titre indicatif mais non limitatif, en référence aux dessins annexés sur lesquels :
- les figures 1A et 1B sont des vues partielles très schématiques illustrant les différentes couches du filtre selon l'invention ;
- les figures 2A et 2B sont des vues partielles d'une variante de réalisation du filtre des figures 1A et 1B ;
- les figures 3 à 6 illustrent différents processus de réalisation du filtre selon les figures précédentes ;
- la figure 7 illustre une vue en perspective éclatée d'un dispositif de purification de l'air de l'habitacle d'un véhicule automobile selon l'invention ;
- la figure 8 est une vue en élévation du dispositif de purification de l'air de la figure 7 ;
- la figure 8A est une vue en coupe selon le plan A-A de la figure 8 ;
- la figure 8b est une vue en coupe selon le plan B-B de la figure 8 montrant des lobes d'émissions lumineuses ;
- la figure 8C est une vue en coupe selon le plan C-C de la figure 8 ;
- la figure 9 est une vue en coupe transversale d'une lampe tubulaire de la figure 8 ;
- la figure 10 illustre très schématiquement un dispositif selon l'invention comportant une source lumineuse en amont du filtre ;
- les figures 11A et 11B illustrent une variante d'un dispositif de purification de l'air selon l'invention ;
- les figures 12A à 12D illustrent très schématiquement différentes variantes de réalisation du dispositif de purification d'air comportant des lampes tubulaires ;
- les figures 13A à 13E illustrent très schématiquement différentes variantes de réalisation du dispositif de purification d'air comportant des lampes plates ;
- les figures 14A à 14E illustrent très schématiquement différentes variantes de réalisation du dispositif de purification d'air comportant des diodes électroluminescentes ;
- la figure 15 illustre très schématiquement un dispositif de purification de l'air comportant un ioniseur ;
- la figure 16 illustre une installation de climatisation pour un véhicule automobile comportant un dispositif de purification de l'air, selon l'invention, entre un groupe moto-ventilateur et un radiateur ;
- la figure 17 illustre un dispositif de purification de l'air selon la figure 16 ;
- la figure 18 illustre une installation de climatisation pour un véhicule automobile comportant un dispositif de purification de l'air, selon l'invention, monté à l'entrée d'air de l'installation ; et
- la figure 19 est un diagramme comparant l'efficacité dans le temps du dispositif de purification de l'air selon l'invention par rapport à des filtres classiques.

### Description détaillée des modes de réalisation

Le principe de l'invention repose sur l'association nouvelle des deux techniques connues de filtration de gaz que sont la filtration par adsorption et la filtration par photocatalyse.

En effet, un adsorbant tel que le charbon actif adsorbe les polluants instantanément en les piégeant dans ses pores sans les détruire.

Ensuite l'agent photocatalyseur, par exemple du dioxyde de titanium «TiO2», désorbe les molécules du polluant piégées sur la surface de l'adsorbant en les détruisant grâce à des réactions chimiques d'oxydation réduction.

Cela permet de régénérer l'adsorbant et par conséquent d'augmenter la durée de vie du filtre pour atteindre environ celle du véhicule.

Dans le cas, où ces réactions chimiques ne sont pas complètes, les sous-produits seront piégés par l'adsorbant et seront décomposés ultérieurement par l'agent photocatalyseur.

L'agent photocatalyseur détruit définitivement les polluants mais son action est lente, d'où l'intérêt de l'associer à un adsorbant afin que les polluants soient piégés instantanément.

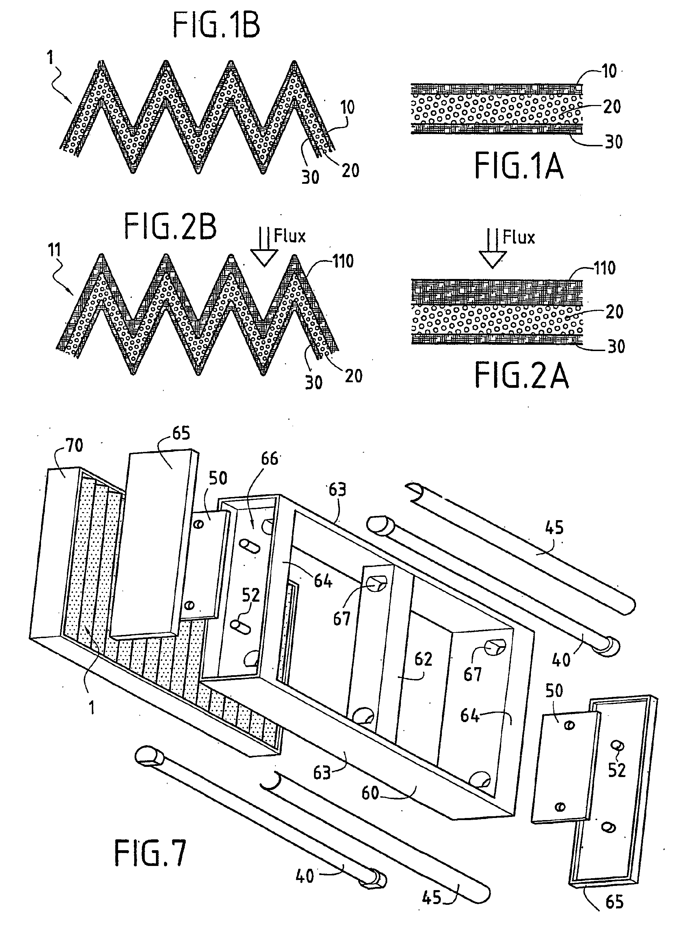

Comme le montrent de façon très schématique les figures 1A et 1B, un filtre selon l'invention est composé de trois couches. La première est une couche 10 de revêtement en fibres non tissées. La seconde est une couche 20 de purification de l'air recouvrant la première couche et constituée d'un agent photocatalyseur 22 intimement associé à des grains d'un adsorbant 24. La troisième est également une couche 30 de revêtement en fibres non tissées recouvrant la seconde couche 20. En outre, cette troisième couche 30 de revêtement peut contenir un agent photocatalyseur afin d'augmenter l'efficacité du filtre.

Les couches de revêtement peuvent être en base PP, PET, PA ou PTFE. A titre d'exemple, l'épaisseur d'une couche de revêtement est comprise entre 0,1 mm et 2 mm.

L'adsorbant peut être composé de charbon actif, de zéolite, d'un mélange des deux ou d'un tout autre adsorbant.

Le charbon actif est par exemple, constitué de grains de dimensions de l'ordre de 0,5 mm à 2 mm. Ces grains sont poreux avec des micropores de dimensions de l'ordre de 0,2 nm à 2 nm et des mesopores de dimensions de l'ordre de 2nm à 50 nm.

En outre, le charbon actif est étendu entre les couches de revêtement avec une masse surfacique comprise entre 150 g/m2 et 450 g/m2.

L'agent photocatalyseur 22 peut par exemple être composé de d'oxyde de titanium « TiO2 » principalement de forme anatase ou d'un tout autre oxyde métallique ayant la propriété de photocatalyse.

L'oxyde de titanium « TiO2 » est sous forme de poudre constitué de particules de dimensions de l'ordre de 40 nm à 500 nm.

Par conséquent, le type de particules de l'oxyde de titanium et leur proportion en masse par rapport au charbon actif est choisie de sorte que les pores de ce dernier ne soient pas obstruées. Ce rapport en masse est par exemple, compris entre 1% et 20%.

L'ensemble de ces trois couches est plissé en accordéon pour faire apparaître des ondulations ou plis afin d'augmenter la surface utile du filtre et diminuer la perte de charge.

En outre, pour filtrer des particules contenues dans l'air, un filtre à particules classique est monté en amont du filtre purificateur 1 par rapport au flux d'air.

Les figures 2A et 2B illustrent une variante de réalisation du filtre. Selon cette variante, la première couche 110 de revêtement est un filtre à particules formant avec les deux autres couches un filtre 11 dit « combiné ». La première couche 110 est destinée à filtrer les particules présentes dans le flux d'air à purifier, tandis que la seconde couche 20 filtre les gaz.

Dans ce cas, le filtre doit être disposé de manière à ce que cette première couche 110 soit en amont de la troisième couche 30 par rapport au flux d'air (flux).

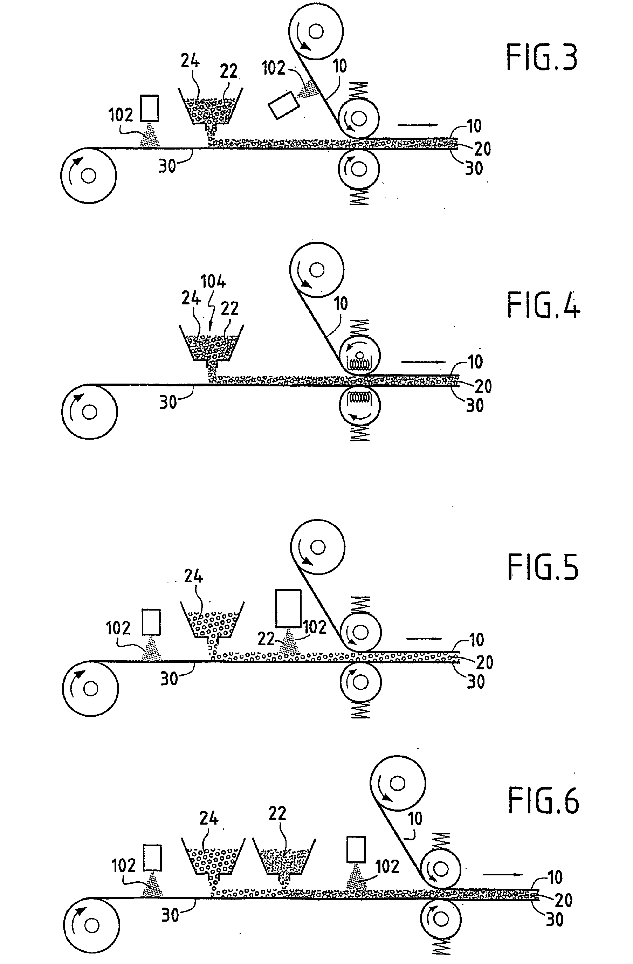

Conformément à l'invention, les figures 3 à 6, illustrent différents modes de réalisation du filtre selon les figures 1A à 2B.

Dans ces différents modes, un agent photocatalyseur 22 (par exemple du TiO2) est intimement associé à des grains d'un adsorbant 24 (par exemple du charbon actif) et le mélange ainsi formé est déposé entre deux couches 10 ou 110 et 30 de revêtement en fibres non tissées.

La figure 3 illustre un premier mode de réalisation, où la poudre du TiO2 est d'abord mélangée à des grains de charbon actif selon les proportions citées plus haut.

Le mélange, ainsi formé est étalé par exemple sur la troisième couche de revêtement ou voile 30 après avoir déposé un adhésif 102, connu en soi, par exemple sous forme de spray sur les faces internes des deux couches de revêtement. En variante, l'adhésif peut être déposé directement sur le mélange. L'adhésif 102 est par exemple de l'Ethylène Vinyl Acétate « EVA » ou du Polyéthylène « PE ».

Ensuite, on applique une pression à froid ou à chaud, par exemple par des rouleaux compresseurs, sur l'ensemble des trois couches ainsi formé, afin de les consolider ensemble.

Les couches de revêtement ou voiles 10, 30 sont réalisés d'une manière classique, par exemple par liage à jet d'eau plus une consolidation mécanique, ou par voie fondue, ou par voie papetière.

Le processus de la figure 3, peut être modifié de la façon illustrée par la figure 4, où un liant 104, connu en soi, par exemple EVA ou PE est ajouté au mélange et où les trois couches sont soudées ensemble par application d'une pression à chaud au niveau des rouleaux compresseurs.

Dans les exemples des figures 5 et 6, le processus reste similaire à précédemment, sauf que l'oxyde de titanium et le charbon actif sont déposés séparément.

Dans le cas de la figure 5, l'oxyde de titanium 22 est mélangé à un adhésif 102 avant d'être pulvérisé ou déposé sur le charbon actif 24 qui lui, a déjà été déposé sur la troisième couche de revêtement 30. Tandis que, dans le cas de la figure 6, le charbon actif, l'oxyde de titanium et l'adhésif sont déposés séparément sur la troisième couche de revêtement 30.

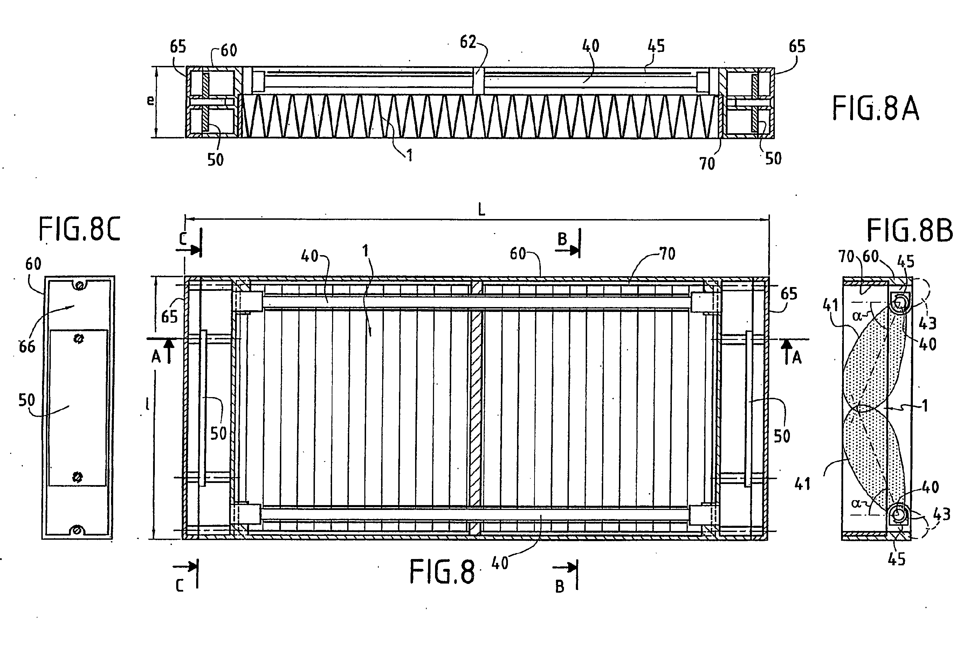

La figure 7, illustre une vue en perspective éclatée d'un dispositif de purification de l'air de l'habitacle d'un véhicule automobile selon l'invention.

Ce dispositif comprend un filtre 1 selon les modes de réalisations décrits plus haut, maintenu dans un support cadre 70. Le cadre 70 est par exemple, de forme rectangulaire dont la surface définit une surface apparente du filtre 1.

Le filtre 1 est globalement rectangulaire et est plissé en accordéon ayant par exemple, des plis en forme de V afin d'augmenter la surface utile du filtre et diminuer la perte de charge.

De façon connue, le cadre filtre 70 peut être en mousse, en matière plastique rigide ou en fibres non tissées de la même matière que la première et la troisième couche du filtre 1. En outre, le cadre filtre 70 peut être réalisé de façon connue par surmoulage ou monté par collage à chaud ou par soudage miroir.

Aucun additif n'est nécessaire dans les deux derniers procédés et par la suite, on appellera filtre 1 le filtre avec son cadre 70.

Un cadre externe 60 épouse sensiblement le cadre support 70 du filtre 1. Le cadre externe est par exemple en matière plastique.

Le cadre externe 60 est constitué de deux côtés longitudinaux 63 reliés par deux côtés transversaux 64 et peut comporter en outre, une traverse de renforcement centrale 62.

Les côtés transversaux 64 et éventuellement la traverse centrale 62 comportent des trous 67 destinés à recevoir une ou plusieurs lampes tubulaires 40 (deux lampes dans l'exemple de la figure 7) afin d'irradier la surface du filtre 1.

Les côtés transversaux 64 présentent des logements 66 destinés à accueillir des transformateurs (non représentés) montés sur des plaques 50. En effet, chaque plaque comporte des trous qui coopèrent avec des ergots ou plots 52 formés dans le logement (voir les figures 7 et 8C). Les plaques peuvent être maintenues en place par un tout autre système de fixation.

Par ailleurs, les transformateurs sont protégés par des couvercles 65 qui se fixent sur les bords des logements 66.

Les transformateurs permettent de délivrer une tension alternative aux lampes 40. A titre d'exemple, chaque transformateur convertit une tension continue de 12V délivrée par la batterie du véhicule en une tension alternative de 1,5 kV à 5 kV d'une fréquence comprise entre 30 kHz et 80 kHz.

Ces transformateurs sont en contact avec les lampes par l'intermédiaire de liaisons ou fils électriques (non représentés). Cependant, ils peuvent être en contact électrique direct avec les lampes, afin d'éliminer toute perturbation par rayonnement électromagnétique.

Chaque lampe peut être alimentée par un transformateur indépendant ou d'une manière similaire toutes les lampes peuvent être alimentées par un transformateur équivalent unique.

Eventuellement, chaque lampe est munie d'un réflecteur en respectant un entrefer lampe-réflecteur d'au moins 1mm. Ces réflecteurs peuvent être de section circulaire (figure 7), parabolique, angulaire ou linéaire, en matériau réfléchissant, par exemple en aluminium poli ou en inox.

Ainsi, comme il est illustré sur les figures 8 à 8C, le filtre 1 encadré par son support 70, les lampes 40, les réflecteurs 45, et les plaques support des transformateurs 50 sont tous montés d'une manière compacte dans le cadre externe 60 pour former un dispositif qui, lorsqu'il est disposé de manière à être traversé par un flux d'air pollué, sert à purifier cet air des polluants gazeux.

Le dispositif selon les figures 8 à 8C est un ensemble fonctionnel compact et est par conséquent montable et démontable aisément.

A titre d'exemple, le cadre externe 60 comporte une longueur (L) comprise entre 200 mm et 500 mm, une largeur (I) comprise entre 100 mm et 300 mm et une épaisseur (e) comprise entre 20 mm et 60 mm.

Dans l'exemple de la figure 8, la distance séparant les deux lampes 40 est de l'ordre de 80mm à 120mm et la distance moyenne entre une lampe et le filtre 1 est de l'ordre de grandeur de 2mm à 30mm, et de préférence de l'ordre de 2mm à 10mm.

En outre, une lampe 40 assure une intensité lumineuse par unité de surface comprise entre 0,5 mW/cm2 et 10 mW/cm2 à une distance d'environ 10 mm,

Ces lampes sont écologiques, sans mercure ni plomb et émettent une lumière d'une longueur d'onde comprise entre 200 nm et 400 nm, autrement dit, des UV de type A, B et C, appropriés pour vaincre la bande interdite d'une molécule de TiO2 afin de permettre une action photocatalytique efficace.

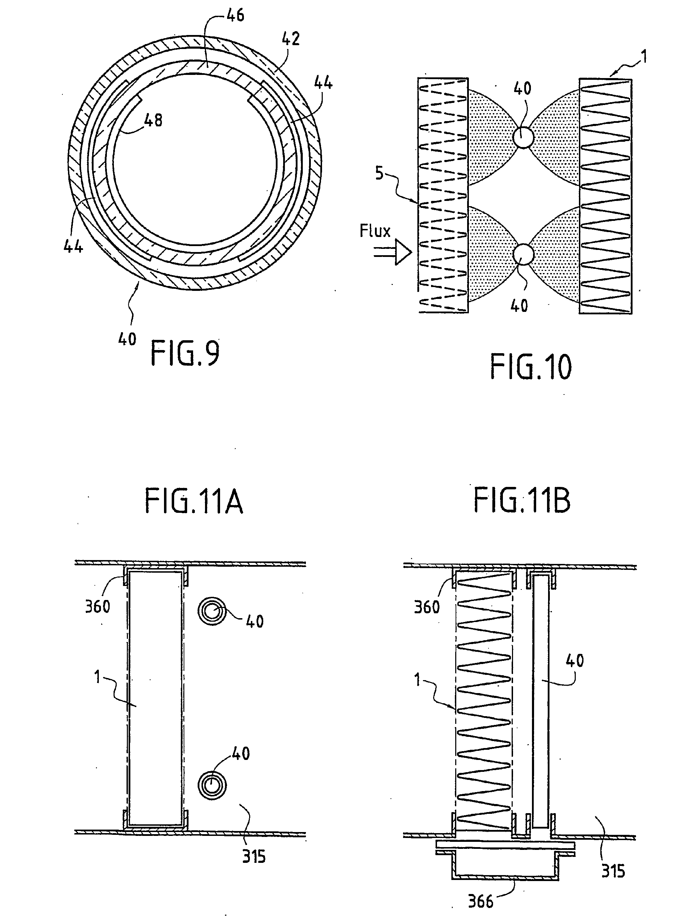

La figure 9 illustre une vue en coupe transversale d'une lampe tubulaire 40. La lampe 40 comporte un tube de verre 46, en quartz renfermant du Xénon et entouré par deux électrodes 44.

Le tube en verre 46 est protégé par un tube externe 42 de protection en PET ou PTFE.

Eventuellement, une couche en phosphore 48 recouvre la face intérieure du tube en verre 46 afin de transformer les rayons UVC en rayons UVA et/ou UVB.

La topologie de la lumière émise par une lampe tubulaire 40 présente un lobe d'émission primaire 41 (voir figure 8B) et un lobe d'émission secondaire 43 représenté en pointillé sur la figure 8B.

Afin d'avoir une efficacité de purification maximale, le lobe d'émission primaire 41 est orienté de sorte à irradier la majorité de la surface utile du filtre 1, c'est-à-dire la surface le plus en contact avec le flux d'air en tenant compte d'un effet de bord qui diminue le flux d'air sur les bords du filtre 1.

Dans l'exemple de la figure 8B, deux lampes 40 sont utilisées, et compte tenu des dimensions et des distances entre les éléments du dispositif explicité plus haut, les lobes primaires 41 de deux lampes 40 sont dirigés vers le centre du filtre 1, de sorte que la médiane du lobe primaire 41 fait un angle α de 35° à 50° par rapport à une perpendiculaire entre la lampe associée 40 et un plan moyen du filtre 1.

Le lobe d'émission secondaire d'une lampe peut par ailleurs, être réfléchi vers la surface utile du filtre 1 par un réflecteur 45, augmentant ainsi, l'efficacité de la radiation du filtre.

La source lumineuse irradiant la surface du filtre, peut être disposée en aval ou en amont du filtre par rapport au flux d'air traversant le dispositif de purification d'air.

La figure 10 montre que dans le cas où la source lumineuse, par exemple deux lampes tubulaires 40, est en amont du filtre à gaz 1, il convient de disposer un filtre à particules 5 en amont de la source lumineuse 40.

Avantageusement, le filtre à particules 5 peut contenir un agent photocatalyseur irradié par les lampes 40, augmentant ainsi l'efficacité de purification d'air.

De plus, dans cette disposition, l'intensité lumineuse des lampes 40 est utilisée de façon optimale car grâce à la réflexion des rayons UV par les filtres 1 et 5, un plus grand nombre de photons sont absorbés par l'agent photocatalyseur.

Les figures 11A et 11B montrent que le dispositif de purification de l'air peut être monté dans un conduit de distribution d'air 315 d'une installation de climatisation sans l'intermédiaire du cadre 60.

En effet, le filtre 1, les lampes 40 et éventuellement les réflecteurs sont montés directement dans des logements 360 disposés dans le conduit 315.

En outre le ou les transformateurs sont montés dans un logement transformateur 366 intégré à l'extérieur du conduit 360, hors le flux d'air, diminuant ainsi l'encombrement et donc la perte de charge.

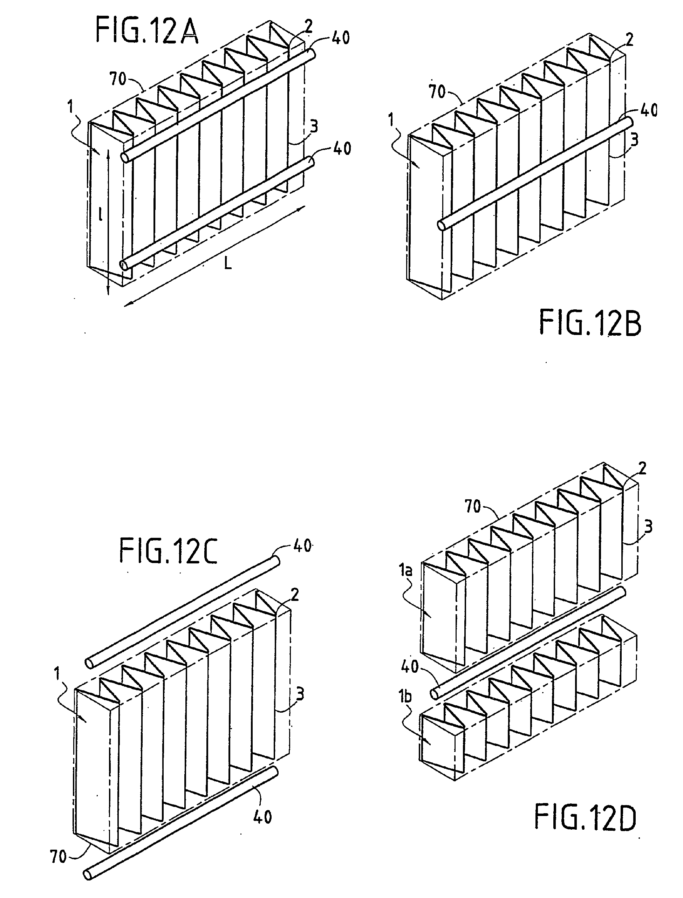

Les figures 12A à 12D illustrent très schématiquement différentes variantes de réalisation du dispositif de purification d'air comportant des lampes tubulaires.

La figure 12A schématise le mode de réalisation illustré par les figures 7 à 11B.

Le filtre 1, étant plissé en accordéon, comporte des crêtes 2 dont les génératrices 3 forment deux plans sensiblement parallèles et perpendiculaires au flux d'air.

Selon l'exemple de la figure 12A, les deux lampes tubulaires sont disposées en aval ou en amont des plans du filtre perpendiculairement à la direction des génératrices 3.

De préférence, les côtés les plus longs du filtre 1 sont perpendiculaires aux génératrices 3 et les deux lampes présentent des longueurs sensiblement égales aux côtés longs L du filtre 1. En outre, les lampes sont espacées d'une distance égale environ à la moitié de la longueur I des autres côtés du filtre.

Selon la figure 12B, le filtre 1 est irradié par une lampe tubulaire 40 unique montée environ au milieu du filtre.

La figure 12C, illustre un dispositif de purification d'air comportant deux lampes tubulaires 40 disposées chacune de part et d'autre du côté long du filtre 1, perpendiculairement à la direction des génératrices 3, et irradiant le filtre dans la direction desdites génératrices.

Le filtre illustré par la figure 12D comprend deux parties la et 1b, séparées selon une section sensiblement perpendiculaire à la direction des génératrices 3, de sorte qu'une lampe tubulaire 40 est disposée entre ces deux parties la et 1b, parallèlement à ladite section, et irradiant le filtre dans les deux sens de la direction des génératrices 3.

Bien entendu, le nombre de lampes montées dans le dispositif de purification de l'air pourra être différent de celui illustré sur les figures 12A à 12D.

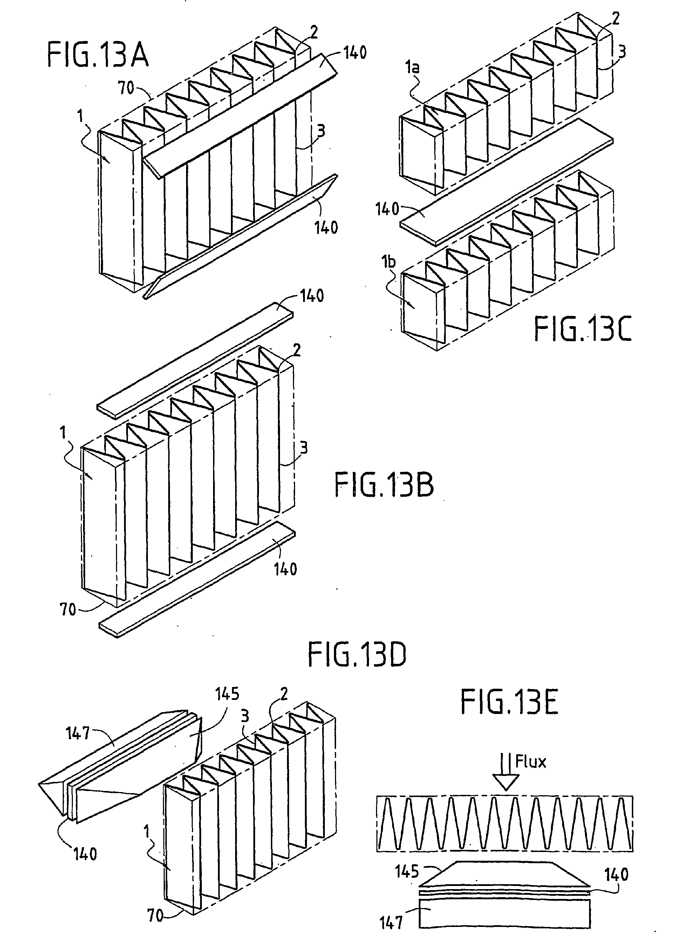

Les figures 13A à 13E illustrent très schématiquement différentes variantes de réalisation du dispositif de purification d'air comportant des lampes plates 140.

La lampe plate 140 comprend un tube plat ayant deux côtés sensiblement parallèles en verre renfermant du Xénon. L'épaisseur du verre est de l'ordre de 1mm à 3mm. Des électrodes en argent ou en tungstène sont réalisées en sérigraphie, comme pour un circuit imprimé, sur un des côtés du tube.

Ces électrodes sont recouvertes d'une couche isolante en SiO2 et Al203 afin d'empêcher ou de limiter les pertes de rayons UV. En outre, l'intérieur de la lampe plate est recouvert de quelques couches moléculaires de phosphore.

Les lampes plates 140 de la figure 13A, sont montées de la même manière que les lampes tubulaires 40 de la figure 12A et sont orientées de sorte à irradier la majorité de la surface utile du filtre 1.

En variante, une seule des deux lampes plates 140 de la figure 13A peut être montée afin d'irradier le filtre 1.

De même, les lampes plates 140 des figures 13B et 13C, sont montées de la même manière que les lampes tubulaires 40 des figures 12B et 12C respectivement.

On notera qu'une seule des deux lampes plates 140 de la figure 13B peut suffire pour irradier ce filtre 1.

Les figures 13D et 13E illustrent très schématiquement une lampe plate 140 unique montée environ au milieu du filtre 1. Dans ce cas, un prisme 145 ayant une forme aérodynamique est monté en amont de la lampe plate 140.

D'une part, le prisme 145 homogénéise l'irradiation du filtre 1 en diffractant la lumière de la lampe plate et d'autre part, grâce à sa forme aérodynamique, diminue la turbulence et le bruit.

Pour une meilleure fluidité du flux d'air, un support 147 aussi d'une forme aérodynamique est monté en aval de la lampe plate.

La figure 13E montre que l'ensemble formé de la lampe plate 140, le prisme 145 et le support 147 est monté en aval du filtre 1 par rapport au flux d'air.

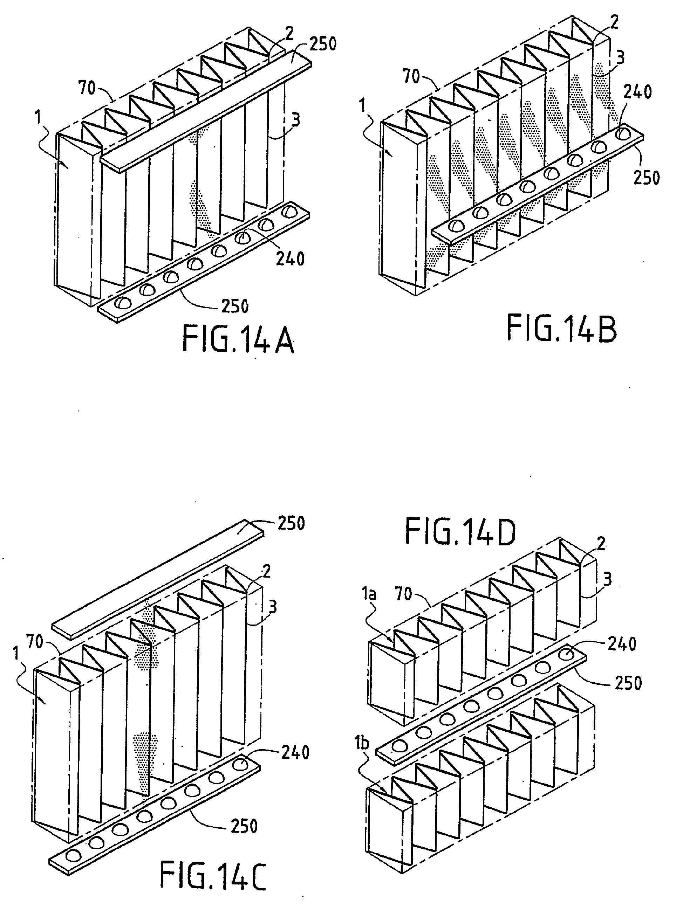

Les figures 14A à 14E illustrent très schématiquement différentes variantes de réalisation du dispositif de purification d'air comportant des diodes électroluminescentes 240. Dans ce cas les transformateurs peuvent être omis.

Selon les figures 14A à 14D une pluralité de diodes électroluminescentes 240 est montée sur une plaque 240.

En effet, selon les figures 14A et 14C, deux plaques 250 sont montées de la même manière que les lampes tubulaires 40 des figures 11A et 11C respectivement. Les diodes électroluminescentes 240 sont montées sur un côté de chaque plaque 240 et sont orientées de sorte à irradier la majorité de la surface utile du filtre 1.

Dans les exemples des figures 14B et 14D, les diodes électroluminescentes 240 sont montées sur les deux côtés de la plaque 240. Par ailleurs, ces plaques sont montées de la même manière que les lampes tubulaires 40 des figures 12B et 12D respectivement.

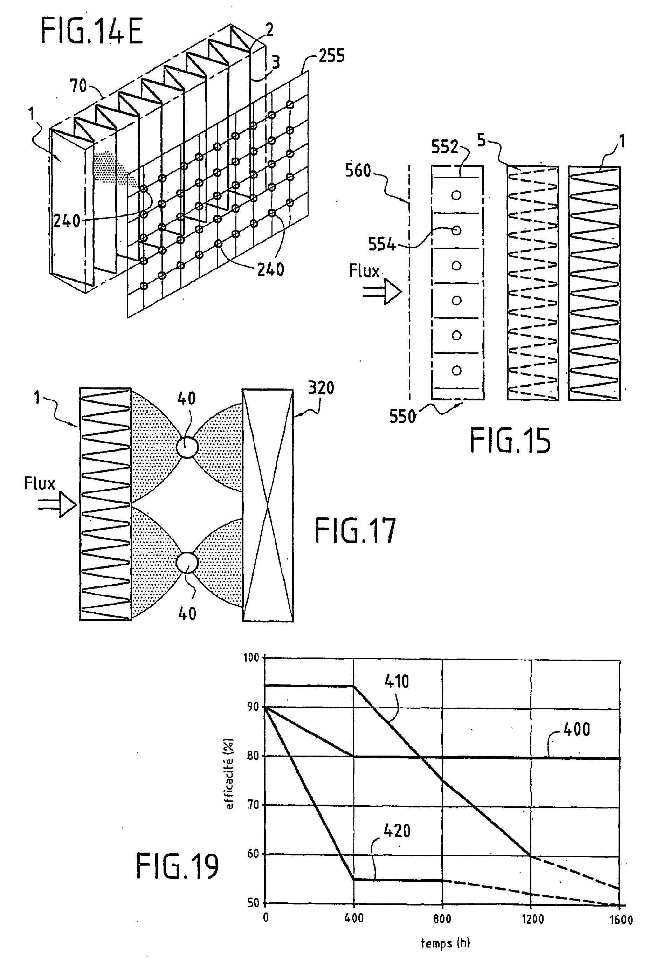

Selon la figure 14E, une pluralité de diodes électroluminescentes 240 est montée sur les noeuds d'une grille 255, disposé parallèlement aux plans définis par les générateurs, de façon à permettre le passage du flux d'air.

On notera que les particules dans l'air sont filtrées par un filtre à particules classique, par exemple en fibres non tissées, monté en amont du dispositif de purification d'air.

Ce filtre à particules peut être combiné avec le filtre à gaz comme illustré sur les figures 2A et 2B ou séparé de lui. L'intérêt de séparer le filtre à particules du filtre à gaz réside dans le fait que leur durée de vie n'étant pas la même, il est ainsi possible de les remplacer séparément.

Dans les deux cas, et afin d'augmenter l'efficacité de filtration, un ioniseur peut être monté en amont du filtre à particules comme illustré sur la figure 15.

L'ioniseur comprend une pluralité de plaques conductrices 552 parallèles disposées en alternance avec des fils électriques 554 de faible diamètre. Les fils sont alimentés au moyen d'un courant alternatif haute tension d'environ 5 kV produit par un module d'alimentation (non représenté) et les plaques sont reliées à la masse.

En amont de l'ioniseur 550 est montée de préférence une grille 560 qui filtre les grosses particules. Les particules en aval de la grille 560 sont ionisées par l'ioniseur 550 et sont collectées par le filtre à particules 5.

Par ailleurs, un même circuit électronique (non représenté) peut être utilisé pour alimenter l'ioniseur 500 et les lampes irradiant le dispositif de purification d'air.

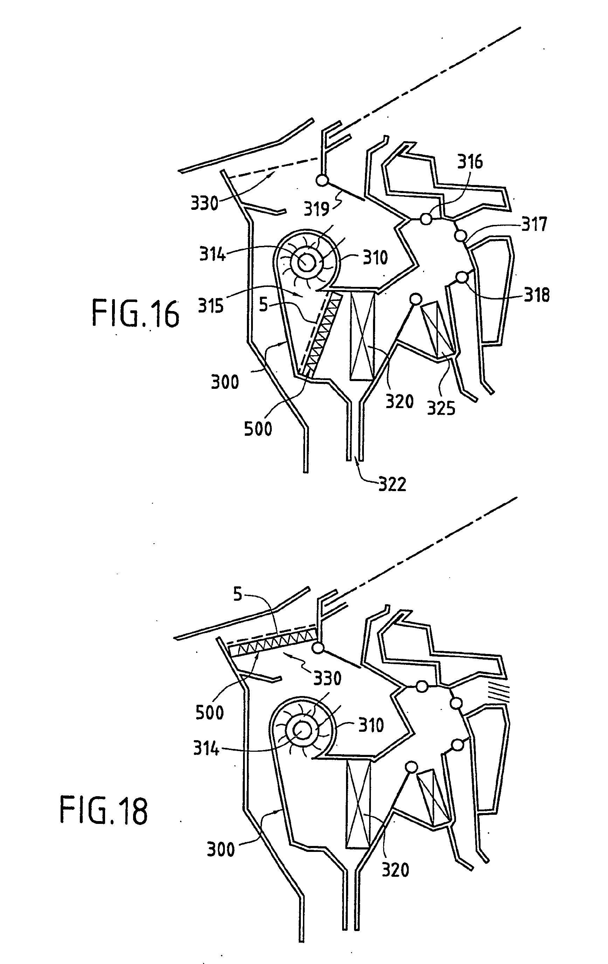

Les figures 16 à 18 illustrent des exemples d'installations de climatisation 300 pour des véhicules automobiles comportant un dispositif de purification de l'air selon l'invention.

L'installation de la figure 16 comporte, de façon bien connue, un groupe moto-ventilateur ou pulseur 310, délivrant un flux d'air dans un conduit de distribution d'air 315. Dans ce dernier, sont disposés un évaporateur 320 d'un circuit de réfrigération (lorsque la fonction climatisation d'air est présente), un radiateur échangeur de chaleur à liquide 325 parcouru par le liquide de refroidissement du moteur du véhicule. De façon connue, un conduit d'écoulement de condensation 322 est disposé sous l'évaporateur 320.

En aval du radiateur 325, le conduit d'air 315 distribue l'air vers des bouches de sortie s'ouvrant dans l'habitacle du véhicule. La distribution et le mixage éventuel de l'air se font à l'aide de volets commandés. Un volet 316 contrôle la sortie dégivrage, un autre 317 contrôle la sortie aération et un troisième volet 318 contrôle la sortie pieds.

L'air circulant dans le conduit provient soit de l'extérieur du véhicule, soit de son habitacle (recyclage intérieur) selon la position d'un volet 319 monté dans la planche de bord du véhicule. L'air est admis dans le conduit 315 à partir d'une ouverture 314 disposée au fond d'un boîtier de volute du pulseur 310.

Le dispositif de purification de l'air 500 selon l'invention est monté dans le conduit 315 entre le groupe moto-ventilateur 310 et l'évaporateur 320. Ainsi, l'air est constamment purifié qu'il soit en recyclage intérieur ou en provenance de l'extérieur du véhicule. Eventuellement, un filtre à particules 5 et/ou un ioniseur (fig. 15) est monté en amont du dispositif 500 par rapport à l'écoulement de l'air.

Le dispositif 500, en ayant un volume réduit et en étant traversé par un fort débit d'air d'environ 450 m³/h,, un seul passage d'air suffit pour purifier l'air avec une grande efficacité d'environ 90% (voir figure 18).

Comme l'illustre la figure 17, il est possible d'utiliser l'évaporateur comme un support d'un agent photocatalyseur et de le faire irradier à partir des sources lumineuses montées en aval du dispositif de purification d'air 500. On notera que dans le cas où des lampes tubulaires 40 sont utilisées, il suffit d'omettre les réflecteurs 45 afin que l'évaporateur soit irradié par la lumière des lobes d'émissions secondaires 43.

Ainsi, l'activation du catalyseur en surface de l'évaporateur permet la destruction et/ou la non-prolifération des micro-organismes se développant sur cette surface réduisant considérablement les mauvaises odeurs.

La figure 18 est une variante où le dispositif de purification de l'air 500 est monté dans l'entrée d'air 330 dans un flux direct en provenance de l'extérieur du véhicule. Le dispositif de purification de l'air 500 est protégé de la pluie et des grosses particules de façon bien connue, par un système de chicanes non représenté.

Un filtre à particules 5 et/ou ioniseur (fig. 15) peut aussi être monté en amont du dispositif 500.

Dans une variante de réalisation, le dispositif de purification de l'air selon l'invention peut être monté dans un boîtier comportant un pulseur compact installé dans l'habitacle même du véhicule automobile.

La figure 19, montre des courbes comparant l'efficacité dans le temps du dispositif de purification de l'air selon l'invention par rapport à des filtres classiques. A titre d'exemple, le polluant choisi afin de faire cette comparaison est le toluène. La courbe 400, représente le dispositif de purification de l'air selon l'invention, la courbe 410 représente un filtre de l'état de l'art constitué de plusieurs lits de charbon actifs et la courbe 420 représente un filtre combiné plissé classique.

Par ailleurs, on rappellera que, du fait de son épaisseur et de sa constitution, le filtre à lits de charbon actifs présente trop de perte de charge vis-à-vis du flux d'air le traversant.

La figure 19, montre que durant les premières quatre cents heures (correspondant à environ 20 000 km), le filtre à lits de charbon actifs (courbe 410) est le plus efficace. Durant la même période, l'efficacité du filtre classique (courbe 420) chute considérablement, tandis qu'une légère décroissance d'efficacité est constatée pour le dispositif selon l'invention (courbe 400).

Par la suite, l'efficacité du dispositif selon l'invention (courbe 400) reste sensiblement stable jusqu'à 1600 heures (correspondant à environ 80 000 km). En revanche, celle du filtre à lits de charbon actifs (courbe 410) décroît assez rapidement et n'est pratiquement, plus efficace au bout de 1200 heures quant à celle du filtre combiné, elle est déjà inefficace dès les premières 400 heures.

On notera que dans le cas où la source lumineuse est en amont du filtre, comme illustré sur la figure 10, l'efficacité du dispositif est accrue d'environ 20%.

Ainsi, le dispositif de purification de l'air selon l'invention augmente la durée de vie du filtre par 4 à 5 fois celle d'un filtre classique à charbon actif.

## Revendications

1. Dispositif de purification de l'air de l'habitacle d'un véhicule comprenant un filtre (1) maintenu dans un cadre (70) définissant une surface apparente du filtre, disposé de manière à être traversé par un flux d'air et une source lumineuse irradiant la surface apparente du filtre, **caractérisé en ce que** le filtre (1 ; 11) comprend une première couche (10 ; 110) de revêtement en fibres non tissées, une seconde couche (20) de purification de l'air recouvrant la première couche et constituée d'un agent photocatalyseur (22) intimement associé à des grains d'un adsorbant (24), et une troisième couche (30) de revêtement en fibres non tissées recouvrant la seconde couche (20), l'ensemble de ces trois couches étant plissé en accordéon pour faire apparaître des ondulations ou plis définissant une surface utile du filtre plus grande que sa surface apparente et dont les génératrices (3) des crêtes (2) forment deux plans sensiblement parallèles et perpendiculaires au flux d'air.

2. Dispositif de purification de l'air selon la revendication 1, **caractérisé en ce que** l'agent photocatalyseur (22) est de l'oxyde de titanium.

3. Dispositif de purification de l'air selon les revendications 1 ou 2, **caractérisé en ce que** l'adsorbant (24) est du charbon actif, de la zéolite ou un mélange des deux.

4. Dispositif de purification de l'air selon la revendication 3, **caractérisé en ce que** le charbon actif a une masse surfacique comprise entre 150 g/m2 et 450 g/m2.

5. Dispositif de purification de l'air selon l'une quelconque des revendications 1 à 4, **caractérisé en ce que** le rapport en masse de l'oxyde de titanium par rapport au charbon actif est compris entre 1% et 20%.

6. Dispositif de purification de l'air selon l'une quelconque des revendications 1 à 5, **caractérisé en ce que** la lumière émise par la source lumineuse comporte une longueur d'onde comprise entre 200 nm et 400 nm.

7. Dispositif de purification de l'air selon l'une quelconque des revendications 1 à 6, **caractérisé en ce que** la source lumineuse et le filtre sont séparés par une distance comprise entre 2 mm et 30 mm.

8. Dispositif de purification de l'air selon l'une quelconque des revendications 1 à 7, **caractérisé en ce que** la source lumineuse, assure une intensité lumineuse par unité de surface comprise entre 0,5 mW/cm2 et 10 mW/cm2, à une distance d'environ 10 mm du filtre.

9. Dispositif de purification de l'air selon l'une quelconque des revendications 1 à 8, **caractérisé en ce que** la source lumineuse, irradiant le filtre (1 ; 11), est constituée d'au moins un moyen d'émission lumineuse (40 ; 140; 240) sans mercure ni plomb.

10. Dispositif de purification de l'air selon la revendication 9, **caractérisé en ce que** le au moins un moyen d'émission lumineuse (40 ; 140 ; 240) est disposé en aval du filtre (1; 11) par rapport au flux d'air et est orienté de sorte à irradier la majorité de la surface utile du filtre.

11. Dispositif de purification de l'air selon la revendication 9, **caractérisé en ce qu'**il comporte en outre un filtre à particules 5 comportant un agent photocatalyseur, le au moins un moyen d'émission lumineuse (40 ; 140 ; 240) est disposé en amont du filtre (1 ; 11) et en aval du filtre à particules 5 par rapport au flux d'air et est orienté de sorte à irradier la majorité de la surface utile du filtre.

12. Dispositif de purification de l'air selon l'une quelconque des revendications 9 à 11, **caractérisé en ce qu'**il comporte deux moyens d'émissions lumineuses (40 ; 140 ; 240).

13. Dispositif de purification de l'air selon l'une quelconque des revendications 1 à 9, **caractérisé en ce que** le filtre comprend deux parties (1a, 1b), séparées selon une section sensiblement perpendiculaire à la direction des génératrices (3), le moyen d'émission lumineuse (40 ; 140 ; 240) étant disposé entre ces deux parties (1a, 1b), parallèlement à ladite section, et irradiant le filtre dans les deux sens de la direction des génératrices (3).

14. Dispositif de purification de l'air selon la revendication 9, **caractérisé en ce qu'**il comporte deux moyens d'émissions lumineuses (40 ; 140 ; 240) disposés chacun de part et d'autre du côté du filtre perpendiculairement à la direction des génératrices (3), et irradiant le filtre dans la direction desdites génératrices.

15. Dispositif de purification de l'air selon la revendication 14, **caractérisé en ce qu'**il comporte un moyen d'émission lumineuse (40 ; 140 ; 240) disposé sur un des côtés.

16. Dispositif de purification de l'air selon l'une quelconque des revendications 9 à 15, **caractérisé en ce que** le moyen d'émission lumineuse est une lampe UV tubulaire (40).

17. Dispositif de purification de l'air selon la revendication 16, **caractérisé en ce que** la lampe UV (40) présente un lobe d'émission primaire orienté de sorte à irradier la majorité de la surface utile du filtre, et un lobe d'émission secondaire réfléchi vers la surface utile du filtre par un réflecteur (45).

18. Dispositif de purification de l'air selon la revendication 16 ou 17, **caractérisé en ce que** chaque lampe UV (40) est alimentée par un transformateur connecté directement à la lampe (40) sans l'intermédiaire de fils électriques.

19. Dispositif de purification de l'air selon l'une quelconque des revendications 9 à 11, **caractérisé en ce que** le moyen d'émission lumineuse comporte deux lampes UV (40) et **en ce que** ces lampes sont alimentées par un transformateur (50) unique.

20. Dispositif de purification de l'air selon l'une quelconque des revendications 16 à 19, **caractérisé en ce que** la ou les lampes (40), le ou les réflecteurs (45), et le ou les transformateurs (50) sont montés dans un cadre externe (60) destiné à recevoir le cadre support (70) du filtre (1 ; 11).

21. Dispositif de purification de l'air selon la revendication 20, **caractérisé en ce que** le cadre externe (60) comporte une longueur comprise entre 200 mm et 500 mm, une largeur comprise entre 100 mm et 300 mm et une épaisseur comprise entre 20 mm et 60 mm.

22. Dispositif de purification de l'air selon l'une quelconque des revendications 9 à 15, **caractérisé en ce que** le moyen d'émission lumineuse est une lampe plate (140).

23. Dispositif de purification de l'air selon la revendication 22, **caractérisé en ce que** la lampe plate 140 comporte un prisme 145 et un support 147 aérodynamique et l'ensemble est monté environ au milieu et en aval du filtre par rapport au flux d'air.

24. Dispositif de purification de l'air selon l'une quelconque des revendications 9 à 15, **caractérisé en ce que** le moyen d'émission lumineuse est une plaque (250) comportant une pluralité de diodes électroluminescentes (240).

25. Dispositif de purification de l'air selon la revendication 9, **caractérisé en ce que** le moyen d'émission lumineuse est une grille (255) comportant une pluralité de diodes électroluminescentes (240) sur ses noeuds, de façon à permettre le passage du flux d'air.

26. Dispositif de purification de l'air selon l'une quelconque des revendications 1 à 25, **caractérisé en ce qu'**il comporte en outre un filtre à particules (5) monté en amont du dispositif.

27. Dispositif de purification de l'air selon la revendication 1, **caractérisé en ce que** la première couche (110) de revêtement est un filtre à particules, et le filtre combiné est disposé de manière à ce que cette première couche (110) soit en amont de la troisième couche (30) par rapport au flux d'air.

28. Dispositif de purification de l'air selon les revendications 26 ou 27, **caractérisé en ce qu'**il comporte en outre un ioniseur (550) monté en amont du filtre à particules (5) ou du filtre combiné (110).

29. Boîtier comportant un pulseur installé dans l'habitacle d'un véhicule, **caractérisé en ce qu'**il comporte un dispositif de purification de l'air selon l'une quelconque des revendications 1 à 28.

30. Installation de climatisation (300) comprenant un groupe moto-ventilateur (310) et un évaporateur (320), délivrant un flux d'air dans un conduit, **caractérisé en ce qu'**il comporte un dispositif de purification de l'air selon l'une quelconque des revendications 1 à 28, monté dans le conduit entre le groupe moto-ventilateur (310) et l'évaporateur (320).

31. Installation selon la revendication 30, **caractérisée en ce que** l'évaporateur (320) sert de support pour un agent photocatalyseur, ce support étant irradié à partir des sources lumineuses montées en aval du dispositif de purification d'air (500).

32. Installation de climatisation (300) comprenant une entrée d'air (330) et un groupe moto-ventilateur (310) délivrant un flux d'air dans un conduit, **caractérisé en ce qu'**il comporte un dispositif de purification de l'air selon l'une quelconque des revendications 1 à 28, monté dans l'entrée d'air (330).

33. Procédé de réalisation d'un filtre pour purifier l'air de l'habitacle d'un véhicule, **caractérisé en ce qu'**on associe intimement un agent photocatalyseur (22) à des grains d'un adsorbant (24) et on dépose le mélange ainsi formé entre deux couches (10, 30) de revêtement en fibre non tissé.

34. Procédé selon la revendication 33, **caractérisé en ce que** l'agent photocatalyseur (22) est de l'oxyde de titanium, l'adsorbant (24) est du charbon actif et **en ce qu'**on élabore un mélange, selon une proportion en masse d'oxyde de titanium par rapport au charbon actif comprise entre 1% et 20%.

35. Procédé selon la revendication 33 ou 34, **caractérisé en ce que** le charbon actif a une masse surfacique comprise entre 150 g/m2 et 450 g/m2.

36. Procédé selon l'une quelconque des revendications 33 à 35, **caractérisé en ce qu'**on applique un adhésif sur les faces internes des deux couches de revêtement (10, 30) avant de déposer le mélange entre elles et on applique une pression à froid sur l'ensemble ainsi formé.

37. Procédé selon l'une quelconque des revendications 33 à 35, **caractérisé en ce qu'**on compose ledit mélange on ajoutant un liant et on applique une pression à chaud sur l'ensemble ainsi formé.

38. Procédé selon l'une quelconque des revendications 33 à 35, **caractérisé en ce qu'**on applique un adhésif (102) sur les faces internes des deux couches de revêtement (10, 30) avant de déposer séparément le titanium et le charbon actif et on applique une pression à froid ou à chaud sur l'ensemble ainsi formé.

## Claims

1. Air purification device for passenger compartment of a vehicle comprising a filter (1) supported in a frame (70) defining an apparent surface of the filter, arranged so that an air flow passes through it, and a light source irradiating the apparent surface of the filter, **characterised in that** the filter (1; 11) comprises a first layer (10; 110) made of non-woven fibres, a second (20) air purification layer covering the first layer and made up of a photocatalyst agent (22) intimately associated with grains of an adsorbent (24), and a third layer (30) made of non-woven fibres covering the second layer (20), all of these three layers being pleated in accordion fashion to create waves or folds defining a useful surface area of the filter larger than the apparent surface area, and for which the generating lines (3) of peaks (2) form two approximately parallel planes perpendicular to the air flow.

2. Air purification device according to claim 1, **characterised in that** the photocatalyst agent (22) is titanium oxide.

3. Air purification device according to either claim 1 or 2, **characterised in that** the adsorbent (24) is active carbon, zeolite or a mixture of the two.

4. Air purification device according to claim 3, **characterised in that** the mass per unit area of active carbon is between 150 g/m2 and 450 g/m2.

5. Air purification device according to any one of claims 1 to 4, **characterised in that** the ratio of the mass of titanium oxide to the mass of active carbon is between 1% and 20%.

6. Air purification device according to any one of claims 1 to 5, **characterised in that** the wavelength of the light emitted by the light source is between 200 nm and 400 nm.

7. Air purification device according to any one of claims 1 to 6, **characterised in that** the light source and the filter are by a distance of between 2 mm and 30 mm.

8. Air purification device according to any one of claims 1 to 7, **characterised in that** the light source creates a light intensity per unit area between 0.5 mW/cm² and 10 mW/cm² at a distance of about 10 mm from the filter.

9. Air purification device according to any one of claims 1 to 8, **characterised in that** the light source irradiating the filter (1; 11) consists of at least one light emission means (40; 140; 240) with no mercury or lead.

10. Air purification device according to claim 9, **characterised in that** the at least one light emission means (40; 140; 240) is located downstream the filter (1; 11) with regard to the airflow and is oriented so as to irradiate most of the useful surface of the filter.

11. Air purification device according to claim 9, **characterised in that** it also comprises a filter (5) comprising a photocatalyst agent, the at least one light emission means (40; 140; 240) is located upstream of the filter (1; 11) with regard to the airflow and downstream of the filter (5) with regard to the airflow and is oriented so as to irradiate most of the useful surface of the filter.

12. Air purification device according to any one of claims 9 to 11, **characterised in that** it comprises two light emission means (40; 140; 240).

13. Air purification device according to any one of claims 1 to 9, **characterised in that** the filter comprises two parts (1a, 1b) separated along a section approximately perpendicular to the direction of the generating lines (3), the light emission means (40; 140; 240) being located between these two parts (1a, 1b) parallel to the said section, and irradiating the filter in the two directions of the generating lines (3).

14. Air purification device according to claim 9, **characterised in that** it comprises two light emission means (40; 140; 240) located on opposite side of the filter perpendicular to the direction of the generating lines (3) and irradiating the filter in the direction of the said generating lines.

15. Air purification device according to claim 14, **characterised in that** it comprises a light emission means (40; 140; 240) on one side.

16. Air purification device according to any one of claims 9 to 15, **characterised in that** the light emission means is a tubular UV lamp (40).

17. Air purification device according to claim 16, **characterised in that** the UV lamp (40) has a primary emission lobe oriented so as to irradiate most of the useful surface of the filter, and a secondary emission lobe reflected towards the useful surface of the filter by a reflector (45).

18. Air purification device according to either claim 16 or 17, **characterised in that** each UV lamp (40) is powered through a transformer connected to the lamp (40) directly without any electrical wires.

19. Air purification device according to any one of claims 9 to 11, **characterised in that** the light emission means comprises two UV lamps (40) and **in that** these lamps are powered through a single transformer (50).

20. Air purification device according to any one of claims 16 to 19, **characterised in that** the lamp(s) (40), the reflector(s) (45) and the transformer(s) (50), are mounted in an external frame (60) into which the support frame (70) of the filter (1; 11) is fitted.

21. Air purification device according to claim 20, **characterised in that** the external frame (60) is between 200 mm and 500 mm long, between 100 mm and 300 mm wide, and between 20 mm and 60 mm thick.

22. Air purification device according to any one of claims 9 to 15, **characterised in that** the light emission means is a flat lamp (140) .

23. Air purification device according to claim 22, **characterised in that** the flat lamp 140 comprises a prism 145 and an aerodynamic support 147 and the assembly is mounted approximately in the middle and downstream of the filter with regard to the airflow.

24. Air purification device according to any one of claims 9 to 15, **characterised in that** the light emission means is a board (250) on which a plurality of light emitting diodes (240) are mounted.

25. Air purification device according to claim 9, **characterised in that** the light emission means is a grille (255) on which a plurality of light emitting diodes (240) are mounted on its nodes so as to allow the air flow to pass through.

26. Air purification device according to any one of claims 1 to 25, **characterised in that** it also comprises a filter (5) mounted upstream of the device.

27. Air purification device according to claim 1, **characterised in that** the first layer (110) is a filter, and the combined filter is arranged such that this first layer (110) is upstream of the third layer (30) with regard to the airflow.

28. Air purification device according to either claim 26 or 27, **characterised in that** it also comprises an ioniser (550) upstream of the filter (5) or of the combined filter (110).

29. Casing comprising a blower installed in the passenger compartment of a vehicle, **characterised in that** it comprises an air purification device according to any one of claims 1 to 28.

30. Air conditioning installation (300) comprising an electric blower (310) and an evaporator (320), blowing an air flow into a duct, **characterised in that** it comprises an air purification device according to any one of claims 1 to 28, mounted in the duct between the electric blower (310) and the evaporator (320).

31. Installation according to claim 30, **characterised in that** the evaporator (320) is used as a support for a photocatalyst agent, this support being irradiated from light sources mounted downstream of the air purification device (500).

32. Air conditioning installation (300) comprising an air inlet (330) and an electric fan (310) blowing an air flow into a duct, **characterised in that** it comprises an air purification device according to any one of claims 1 to 28, mounted in the air inlet (330).

33. Process for manufacturing a filter to purify air in the passenger compartment of a vehicle, **characterised in that** a photocatalyst agent (22) is intimately associated with grains of an adsorbent (24) and the mixture thus formed is deposited between two layers (10, 30) made of non-woven fibres.

34. Process according to claim 33, **characterised in that** the photocatalyst agent (22) is titanium oxide, the adsorbent (24) is active carbon, and **in that** a mixture is produced such that the ratio of the mass of titanium oxide to the mass of active carbon is between 1% and 20%.

35. Process according to either claim 33 or 34, **characterised in that** the mass per unit area of active carbon is between 150 g/m2 and 450 g/m2.

36. Process according to any one of claims 33 to 35, **characterised in that** an adhesive is applied to the two internal faces of the layers (10, 30) before the mixture is deposited between the layers and a pressure is applied to the assembly thus formed when cold.

37. Process according to any one of claims 33 to 35, **characterised in that** the said mixture is created by adding a binder and pressure is applied to the assembly thus formed when hot.

38. Process according to any one of claims 33 to 35, **characterised in that** an adhesive (102) is applied to the internal faces of the two layers (10, 30) before depositing titanium and active carbon separately, and pressure is applied to the assembly thus formed when cold or hot.

## Patentansprüche

1. Vorrichtung zur Reinigung der Luft in der Fahrgastzelle eines Fahrzeugs, einen Filter (1) aufweisend, der in einem die sichtbare Fläche des Filters definierenden Rahmen (70) gehalten wird, derart angeordnet, dass er von einem Luftstrom durchquert wird, und eine die sichtbare Fläche des Filters bestrahlende Lichtquelle, **dadurch gekennzeichnet, dass** der Filter (1; 11) eine erste Überzugsschicht (10; 110) aus Vliesfasern, eine zweite Schicht (20) zur Luftreinigung, welche die erste Schicht bedeckt und aus einem Photokatalysatormittel (22) besteht, das eng mit den Körnern eines Adsorptionsmittels (24) verbunden ist, und eine dritte Überzugsschicht (30) aus Vliesfasern, welche die zweite Schicht (20) bedeckt, aufweist, wobei alle diese drei Schichten als Ziehharmonika gefaltet sind, um Wellen oder Falten zu bilden, die eine Nutzfläche des Filters definieren, die größer ist als seine sichtbare Fläche und deren Kanten (3) der Spitzen (2) zwei Ebenen bilden, die etwa parallel sind und senkrecht zum Luftstrom.

2. Vorrichtung zur Reinigung der Luft nach Anspruch 1, **dadurch gekennzeichnet, dass** das Photokatalysatormittel (22) Titandioxid ist.

3. Vorrichtung zur Reinigung der Luft nach Anspruch 1 oder 2, **dadurch gekennzeichnet, dass** das Adsorptionsmittel (24) Aktivkohle, Zeolith oder eine Mischung aus beiden ist.

4. Vorrichtung zur Reinigung der Luft nach Anspruch 3, **dadurch gekennzeichnet, dass** die Aktivkohle eine flächenbezogene Masse von 150 g/m² bis 450 g/m² hat.

5. Vorrichtung zur Reinigung der Luft nach einem der Ansprüche 1 bis 4, **dadurch gekennzeichnet, dass** das Massenverhältnis des Titandioxids zu der Aktivkohle 1 % bis 20 % beträgt.

6. Vorrichtung zur Reinigung der Luft nach einem der Ansprüche 1 bis 5, **dadurch gekennzeichnet, dass** das von der Lichtquelle ausgestrahlte Licht eine Wellenlänge von 200 nm bis 400 nm hat.

7. Vorrichtung zur Reinigung der Luft nach einem der Ansprüche 1 bis 6, **dadurch gekennzeichnet, dass** die Lichtquelle und der Filter 2 mm bis 30 mm voneinander beabstandet sind.

8. Vorrichtung zur Reinigung der Luft nach einem der Ansprüche 1 bis 7, **dadurch gekennzeichnet, dass** die Lichtquelle bei einem Abstand von etwa 10 mm vom Filter pro Flächeneinheit eine Lichtstärke von 0,5 mW/cm² bis 10 mW/cm² gewährleistet.

9. Vorrichtung zur Reinigung der Luft nach einem der Ansprüche 1 bis 8, **dadurch gekennzeichnet, dass** die den Filter (1; 11) bestrahlende Lichtquelle aus mindestens einem quecksilber- und bleifreiem Lichtemissionsmittel (40; 140; 240) besteht.

10. Vorrichtung zur Reinigung der Luft nach Anspruch 9, **dadurch gekennzeichnet, dass** das mindestens eine Lichtemissionsmittel (40; 140; 240) im Verhältnis zum Luftstrom nach dem Filter (1; 11) angeordnet ist und derart ausgerichtet, dass es den größten Teil der Nutzfläche des Filters bestrahlt.

11. Vorrichtung zur Reinigung der Luft nach Anspruch 9, **dadurch gekennzeichnet, dass** sie außerdem einen Partikelfilter (5) umfasst, der ein Photokatalysatormittel umfasst, wobei das mindestens eine Lichtemissionsmittel (40; 140; 240) im Verhältnis zum Luftstrom vor dem Filter (1; 11) und nach dem Partikelfilter (5) angeordnet und derart ausgerichtet ist, dass es den größten Teil der Nutzfläche des Filters bestrahlt.

12. Vorrichtung zur Reinigung der Luft nach einem der Ansprüche 9 bis 11, **dadurch gekennzeichnet, dass** sie zwei Lichtemissionsmittel (40; 140; 240) umfasst.

13. Vorrichtung zur Reinigung der Luft nach einem der Ansprüche 1 bis 9, **dadurch gekennzeichnet, dass** der Filter zwei Teile (1a, 1b) umfasst, die gemäß einer etwa senkrecht zur Richtung der Kanten (3) liegenden Schnittlinie getrennt sind, wobei das Lichtemissionsmittel (40; 140; 240) zwischen diesen beiden Teilen (1a, 1b) parallel zu der besagten Schnittlinie angeordnet ist und den Filter in beide Richtungen in Richtung der Kanten (3) bestrahlt.

14. Vorrichtung zur Reinigung der Luft nach Anspruch 9, **dadurch gekennzeichnet, dass** sie zwei Lichtemissionsmittel (40; 140; 240) umfasst, die jeweils auf der einen und anderen Seite des Filters senkrecht zur Richtung der Kanten (3) angeordnet sind und den Filter in Richtung der besagten Kanten bestrahlen.

15. Vorrichtung zur Reinigung der Luft nach Anspruch 14, **dadurch gekennzeichnet, dass** sie ein Lichtemissionsmittel (40; 140; 240) umfasst, das auf einer der Seiten angeordnet ist.

16. Vorrichtung zur Reinigung der Luft nach einem der Ansprüche 9 bis 15, **dadurch gekennzeichnet, dass** das Lichtemissionsmittel eine röhrenförmige UV-Lampe (40) ist.

17. Vorrichtung zur Reinigung der Luft nach Anspruch 16, **dadurch gekennzeichnet, dass** die UV-Lampe (40) eine primäre Strahlungskeule aufweist, die derart ausgerichtet ist, dass sie den größten Teil der Nutzfläche des Filters bestrahlt, und eine sekundäre Strahlungskeule, die von einem Reflektor (45) in Richtung der Nutzfläche des Filters reflektiert wird.

18. Vorrichtung zur Reinigung der Luft nach Anspruch 16 oder 17, **dadurch gekennzeichnet, dass** jede UV-Lampe (40) von einem Transformator versorgt wird, der direkt, ohne elektrische Kabel, mit der Lampe (40) verbunden ist.

19. Vorrichtung zur Reinigung der Luft nach einem der Ansprüche 9 bis 11, **dadurch gekennzeichnet, dass** das Lichtemissionsmittel zwei UV-Lampen (40) umfasst und **dadurch**, dass diese Lampen von einem einzigen Transformator (50) versorgt werden.

20. Vorrichtung zur Reinigung der Luft nach einem der Ansprüche 16 bis 19, **dadurch gekennzeichnet, dass** die Lampe oder Lampen (40), der Reflektor oder die Reflektoren (45), der Transformator oder die Transformatoren (50) in einem externen Rahmen (60) montiert sind, der zur Aufnahme des Trägerrahmens (70) des Filters (1; 11) bestimmt ist.

21. Vorrichtung zur Reinigung der Luft nach Anspruch 20, **dadurch, gekennzeichnet, dass** der externe Rahmen (60) 200 mm bis 500 lang, 100 mm bis 300 mm breit und 20 mm bis 60 mm dick ist.

22. Vorrichtung zur Reinigung der Luft nach einem der Ansprüche 9 bis 15, **dadurch gekennzeichnet, dass** das Lichtemissionsmittel eine flache Lampe (140) ist.

23. Vorrichtung zur Reinigung der Luft nach Anspruch 22, **dadurch gekennzeichnet, dass** die flache Lampe (140) ein Prisma (145) und eine aerodynamische Halterung (147) umfasst und die Konstruktion ungefähr in der Mitte und im Verhältnis zum Luftstrom nach dem Filter angebracht ist.

24. Vorrichtung zur Reinigung der Luft nach einem der Ansprüche 9 bis 15, **dadurch gekennzeichnet, dass** das Lichtemissionsmittel eine Platte (250) ist, die eine Vielzahl von Elektrolumineszenzdioden (240) umfasst.

25. Vorrichtung zur Reinigung der Luft nach Anspruch 9, **dadurch gekennzeichnet, dass** das Lichtemissionsmittel ein auf seinen Schnittpunkten eine Vielzahl von Elektrolumineszenzdioden (240) umfassendes Gitter (255) ist, das von dem Luftstrom durchquert werden kann.

26. Vorrichtung zur Reinigung der Luft nach einem der Ansprüche 1 bis 25, **dadurch gekennzeichnet, dass** sie außerdem einen Partikelfilter (5) umfasst, der vor der Vorrichtung angebracht ist.

27. Vorrichtung zur Reinigung der Luft nach Anspruch 1, **dadurch gekennzeichnet, dass** die erste Überzugsschicht (110) ein Partikelfilter ist und der kombinierte Filter derart angeordnet ist, dass sich diese erste Schicht (110) im Verhältnis zum Luftstrom vor der dritten Schicht (30) befindet.

28. Vorrichtung zur Reinigung der Luft nach Anspruch 26 oder 27, **dadurch gekennzeichnet, dass** sie außerdem einen Ionisator (550) umfasst, der vor dem Partikelfilter (5) oder dem kombinierten Filter (110) angebracht ist.

29. Gehäuse, das ein Gebläse umfasst, das in der Fahrgastzelle eines Fahrzeugs installiert ist, **dadurch gekennzeichnet, dass** es eine Vorrichtung zur Reinigung der Luft nach einem der Ansprüche 1 bis 28 umfasst.

30. Klimaanlage (300), die einen Lüftersatz (310) und, einen Verdampfer (320) aufweist, die einen Luftstrom in eine Leitung abgeben, **dadurch gekennzeichnet, dass** sie eine Vorrichtung zur Reinigung der Luft nach einem der Ansprüche 1 bis 28 umfasst, die in der Leitung zwischen dem Lüftersatz (310) und dem Verdampfer (320) angebracht ist.

31. Anlage nach Anspruch 30, **dadurch gekennzeichnet, dass** der Verdampfer (320) als Träger für ein Photokatalysatormittel dient, wobei dieser Träger von Lichtquellen bestrahlt wird, die nach der Vorrichtung zur Reinigung der Luft (500) angebracht sind.

32. Klimaanlage (300), die einen Lufteinlass (330) und einen Lüftersatz (310) aufweist, die einen Luftstrom in eine Leitung abgeben, **dadurch gekennzeichnet, dass** sie eine Vorrichtung zur Reinigung der Luft nach einem der Ansprüche 1 bis 28 umfasst, die in den Lufteinlass (330) eingebaut ist.

33. Verfahren zur Herstellung eines Filters zum. Reinigen der Luft in einer Fahrgastzelle eines Fahrzeugs, **dadurch gekennzeichnet, dass** ein Photokatalysatormittel (22) mit den Körnern eines Adsorptionsmittels (24) eng verbunden und die derart hergestellte Mischung zwischen zwei Überzugsschichten aus. Vliesfasern (10, 30) verbracht wird.

34. Verfahren nach Anspruch 33, **dadurch gekennzeichnet, dass** das Photokatalysatormittel (22) Titandioxid ist, das Adsorptionsmittel (24) Aktivkohle ist und **dadurch**, dass entsprechend einem Mengenverhältnis von 1 % bis 20 % von Titandioxid zu Aktivkohle eine Mischung hergestellt wird.

35. Verfahren nach Anspruch 33 oder 34, **dadurch gekennzeichnet, dass** die Aktivkohle eine flächenbezogene Masse von 150 g/m² bis 450 g/m² hat.

36. Verfahren nach einem der Ansprüche 33 bis 35, **dadurch gekennzeichnet, dass** auf die Innenseiten der beiden Überzugsschichten (10, 30) ein Klebstoff aufgetragen wird, bevor die Mischung zwischen sie verbracht wird, und die derart gebildete Konstruktion kalt gepresst wird.

37. Verfahren nach einem der Ansprüche 33 bis 35, **dadurch gekennzeichnet, dass** die besagte Mischung durch Hinzufügen eines Bindemittels gebildet wird und die derart gebildete Konstruktion warm gepresst wird.

38. Verfahren nach einem der Ansprüche 33 bis 35, **dadurch gekennzeichnet, dass** auf die Innenseiten der beiden Überzugsschichten (10, 30) ein Klebstoff (102) aufgetragen wird, bevor separat das Titan und die Aktivkohle aufgetragen werden und die derart gebildete Konstruktion kalt oder warm gepresst wird.
